Europäisches Patentamt

European Patent Office

Office européen des brevets

(19)

(11) Publication number: **0 326 111**
**A2**

(12) **EUROPEAN PATENT APPLICATION**

(21) Application number: **89101282.5**

(22) Date of filing: **25.01.89**

(51) Int. Cl.⁴: **C07K 17/12 , A61K 39/385 , A61K 39/29**

(30) Priority: **29.01.88 US 149759**

(43) Date of publication of application:
**02.08.89 Bulletin 89/31**

(84) Designated Contracting States:
**DE FR GB IT**

(71) Applicant: **New York Blood Center, Inc.**
**310 East 67 Street**
**New York, New York 10021(US)**

(72) Inventor: **Neurath, Alexander R.**
**230 East 79th Street**
**New York, N.Y. 10021(US)**

(74) Representative: **Patentanwälte Dipl.-Ing. R. Splanemann Dr. B. Reitzner Dipl.-Ing. K. Baronetzky**
**Tal 13**
**D-8000 München 2(DE)**

(54) Peptide derivatives rendered immunogenic when administered with alum as an adjuvant.

(57) A physiologically acceptable immunogenic complex comprising a peptide linked to a non-immunogenic, high molecular weight polysaccharide carrier, the carrier having been converted into an amino derivative (and preferably cross-linked by a dialdehyde), the peptide linked to the carrier being adsorbed onto a physiologically acceptable adjuvant. A vaccine comprising the complex in a physiologically acceptable diluent.

EP 0 326 111 A2

# PEPTIDE DERIVATIVES RENDERED IMMUNOGENIC WHEN ADMINISTERED WITH ALUM AS AN ADJUVANT

## BACKGROUND OF THE INVENTION

### Field of the Invention

The present invention concerns physiologically acceptable immunogenic complexes. More particularly the present invention relates to immunogenic peptide derivatives linked to non-immunogenic high molecular weight carriers, the peptide carrier complex having the property of being adsorbable to alum, a physiologically acceptable adjuvant. Still further, the invention is directed to vaccines comprising such complexes.

### Background of the Invention

Peptides have to be linked to protein carriers or have to be administered in oil-containing adjuvants, for example, complete and incomplete Freund's adjuvant in order to become immunogenic. Such adjuvants are not approved world-wide at the present time for human use. The only adjuvant presently approved for vaccination of humans is alum ($M'Al(SO_4)_3 \bullet 12H_2O$, whre $M'$ is $NH_4^+$ or $K^+$). To realize the potential of synthetic peptides as vaccines for prevention of infections diseases, it is preferred to design peptide derivatives which are immunogenic when administered with alum. Such peptide derivatives have been prepared by linking peptides, particularly synthetic peptides, to immunogenic protein carriers. Such carriers also elicit anti-carrier immune responses which may not be desirable and may also lead to a decreased immune response to the synthetic peptide. For this reason new methods for the preparation of peptide derivatives, based on linking such peptides to non-immunogenic high molecular weight carriers would be extremely desirable.

| DEFINITIONS | | |
|---|---|---|
| Amino Acid Code Words | | |
| D | Asp | aspartic acid |
| N | Asn | asparagine |
| T | Thr | threonine |
| S | Ser | serine |
| E | Glu | glutamic acid |
| Q | Gln | glutamine |
| P | Pro | proline |
| G | Gly | glycine |
| A | Ala | alanine |
| C | Cys | cysteine |
| V | Val | valine |
| M | Met | methionine |
| I | Ile | isoleucine |
| L | Leu | leucine |
| Y | Tyr | tyrosine |
| F | Phe | phenylalanine |
| W | Trp | tryptophane |
| K | Lys | lysine |
| H | His | histidine |
| R | Arg | arginine |
| HBV | | hepatitis B virus |
| HBsAg | | hepatitis B surface antigen |
| DNA | | deoxyribonucleic acid |

## SUMMARY OF THE INVENTION

The present invention relates to a physiologically acceptable immunogenic complex comprising a peptide, e.g., a synthetic peptide, linked to a high molecular weight, non-immunogenic polysaccharide carrier, the carrier having been converted into an amino derivative (and preferably cross-linked by a dialdehyde), the peptide linked to the carrier being absorbed to a physiologically acceptable adjuvant, e.g., alum or aluminum phosphate.

The present invention also concerns vaccines employing an immunologically effective amount of the aforementioned complex with physiologically acceptable diluents.

The present invention is also directed to a method of making the aforementioned complexes. Such method involves introducing amino groups onto a high molecular weight, non-immunogenic polysaccharide carrier, linking a peptide, e.g., a synthetic peptide, to the high molecular weight polysaccharide carrier and adsorbing the resultant peptide linked to said carrier onto a physiologically acceptable adjuvant.

The complexes of the present invention can be made in several ways, however, the first step is adding amino groups to a high molecular weight, non-immunogenic polyssacharide carrier. The polysaccharide may then be crosslinked through the amino groups by contacting the polysaccharide with a dialdehyde. If an uncrosslinked polyssacharide containing amino groups is used, a heterobifunctional agent can be used to link a peptide and thereafter the complex is adsorbed to an adjuvant. Alternatively, if a crosslinked polyssacharide is used several routes can be followed. In one case, the peptide can be directly linked and thereafter the complex can be adsorbed to the adjuvant. Alternatively, lysine or a polyamino amino acid with at least two amino groups can be used to introduce bridges to link a peptide with the use of a crosslinking agent. Still further, cysteine groups can be introduced to the crosslinked carrier and thereafter cysteine-containing peptides can be linked.

## BRIEF DESCRIPTION OF THE DRAWINGS

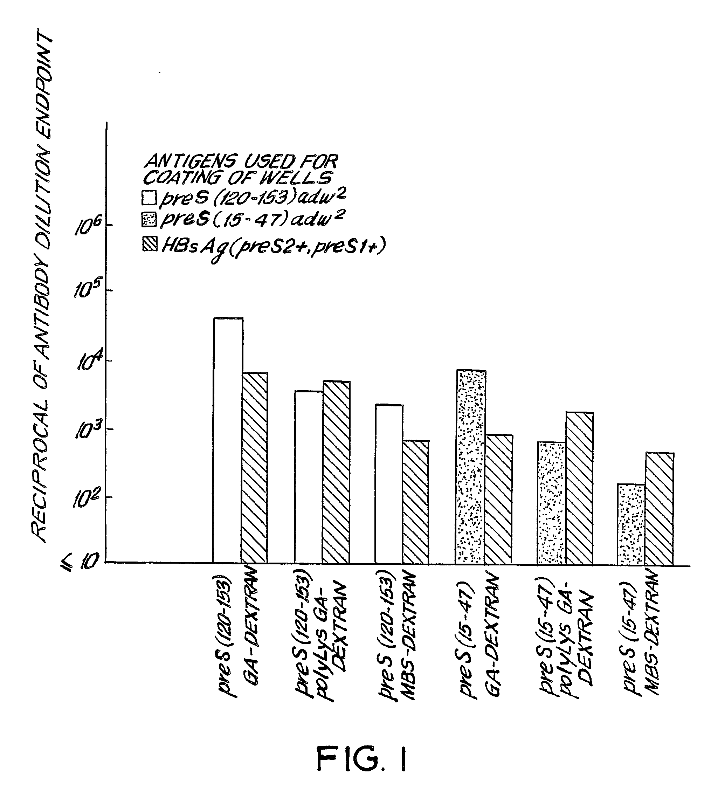

Fig. 1 is a series of bar graphs for different carriers showing the effect of the use of such carriers on the immunogenicity of hepatitis B virus preS2 and preS1 peptides.

## DETAILED DESCRIPTION OF THE INVENTION

The present invention is applicable to any immunogenic synthetic peptide. Of particular interest is the use of the present invention with hepatitis B virus or human immunodeficiency virus (HIV) peptides.

Generally the peptide for use in the invention will have at least 5 amino acids and will have no more than 100 amino acids, preferably no more than 75 amino acids and more preferably no more than 50 amino acids.

The hepatitis B virus (HBV) genome is composed of two DNA strands, namely the long (L) strand and the short (S) strand. The L strand transcript has four open reading frame regions which are termed (S + preS), C, P and X.

The open reading frame region (S + preS) corresponds to the envelope (env) gene of HBV DNA and codes for a family of proteins found in the HBV envelope and in virus related particles.

A schematic representation of the potential translation products of the env gene(s) of HBV DNA is as follows:

Pre-S region          S region

```
|--------------------------------------|  |-----------------------------|
1        12        120              174    175                        400
```

```
|-----------|----------|------------|------|  |------|------------------------|
preS(1)   preS(12)   preS(120)   preS(174)   S(1)                       S(226)
1                                             175                        400
```

S region only:

```
                                           |------------------------------|
                                                                       S(226)
                                                                        400
```

S + preS2 regions:

```
                                 |------------------------------------------|
                                 preS(120)                              S(226)
                                                                         400
```

```
                          |-----------------------------------------------|
                          preS(12)                                   S(226)
                                                                      400
```

S + preS1 + preS2 regions:

```
preS1 region                    preS2 region        S region
|-------------------------------|------------------|-------------------------|
preS(1)                         preS(120)          S(1)                 S(226)
1                                                                       400
```

The numbers in the above schematic refers to amino acids (AA). A translation initiation site at Met 1 exists for the adw$_2$ and adr sybtypes only. The first amino acid for the other sybtypes correspond to position preS 12.

Hereinafter, amino acid sequences corresponding to the preS region (env 1 to 174) are designated with the prefix "preS" and amino acid sequences corresponding to the S region (env 175 to 400) are designated by the prefix "S". In the env gene product representation, the S region spans amino acids 175 to 400, as compared to amino acids 1 to 226 in the "S region only" representation.

In the above schematic, the preS region is defined by amino acid sequence positions preS 1 to amino acid sequence position preS 174. The S region is defined by sequence positions S 1 (amino acid 175 of the open reading frame and adjacent to preS 174) to sequence position S 266 (amino acid 400 of the open reading frame). The S-gene produce (S-protein) consists of this 226 amino acid sequence.

The naturally occurring envelope proteins of hepatitis B virus include the following:

(1) a full length translational product of the env gene of HBV, i.e., for adw$_2$ and adr preS(1-174) + S-(175-400) = 400 amino acids, for ayw, adyw and adw preS(12-174) + S(1-226) = 389 amino acids (env 12-400);

(2) preS(120-174) + S(175-400) = 281 amino acids (env 120-400) = terminal 55 amino acids in the preS region + 226 amino acids comprising the entire S region (the corresponding proteins approximately 33 and 36 kD in size (P33 and P36), and differing from each other in the extent of glycosylation); and

(3) S(1-226) = 226 amino acids, i.e., the entire S region (env 175-400); representing the approximately 22 and 26 kD major constituents of the HBV envelope (GP22 and GP26) in their non-glycosylated and glycosylated forms (the "S-protein").

Preferred preS peptides for use in the present invention include the following:
(1) preS(12-31), all subtypes,
(2) preS(120-145), all subtypes,
(3) preS(32-53), all subtypes,
(4) preS(117-134), all subtypes,
(5) preS(94-117), all subtypes,
(6) preS(153-171), all subtypes,
(7) preS(1-21), all subtypes,

(8) preS(57-73), all subtypes,

(9) preS(1-12), subtype adw2,

(10) preS(1-12), subtype adr,

(11) preS(12-47), all subtypes,

(12) preS(21-47), all subtypes,

(13) preS(120-3153, all subtypes,

(14) preS(132-137), all subtypes,

(15) preS(53-73), all subtypes,

(16) preS(128-139), all subtypes,

(17) preS(15-47), all subtypes.

Non-limiting examples of hepatitis B virus peptides corresponding to the S region of the surface antigen of hepatitis B virus for use in the present invention including the following:

(1)

| 141 | 142 | 143 | 144 | 145 | 146 |
|-----|-----|-----|-----|-----|-----|
| Lys | Pro | Thr | Asp | Gly | Asn, |

(2)

| 138 | 139 | 140 | 141 | 142 | 143 | 144 | 145 | 146 | 147 | 148 | 149 |
|-----|-----|-----|-----|-----|-----|-----|-----|-----|-----|-----|-----|
| Cys | Cys | Thr | Lys | Pro | Thr | Asp | Gly | Asn | Cys | Thr | Cys |

(3)

```
Ser —— Thr —— Gly — Pro — Ser
117                        121
                                       Lys
                                       122
                                        |
                                       Thr
                                        |
                    Ser — Cys  = Cys - Met - Thr
                     |     137   124         |
                    Pro                     Thr
                     |                       |
                    Tyr                     Ala
                     |                       |
                    Met — Ser — Thr — Gly — Gln,
```

| Position | Amino Acid Series |
|----------|-------------------|
| (4) 48-81 | Cys-Leu-Gly-Gln-Asn-Ser-Gln-Ser-Pro-Thr-Ser-Asn-His-Ser-Pro-Thr-Ser-Cys-Pro-Pro-Thr-Cys-Pro-Gly-Thr-Arg-Trp-Met-Cys-Leu-Arg-Arg-Phe-Ile |

(5)   2-16      Glu-Asn-Ile-Thr-Ser-Gly-Phe-Leu-Gly-Pro-
                Leu-Leu-Val-Leu-Gln-Cys

(6)   22-35     Leu-Thr-Arg-Ile-Leu-Thr-Ile-Pro-Gln-Ser-
                Leu-Asp-Ser-Trp-Cys

(7)   38-52     Ser-Leu-Asn-Phe-Leu-Gly-Gly-Thr-Thr-Val-
                Cys-Leu-Gly-Gln-Asn

(8)   47-52     Val-Cys-Leu-Gly-Gln-Asn

(9)   95-109    Leu-Val-Leu-Leu-Asp-Tyr-Gln-Gly-Met-Leu-
                Pro-Val-Cys-Pro-Leu

(10)  104-109   Leu-Pro-Val-Cys-Pro-Leu.

Furthermore peptide analogues of the following non-limiting examples of viruses can be employed in the present invention: influenza hemagglutinin (A/memphis/102/72 strain, A/Eng 1878/69 strain, A/NT/60/68/29c strain, and A/Qu/7/70 strain), fowl plague virus hemagglutinin, vaccinia, polio, rubella, cytomegalovirus, small pox, herpes simplex types I and II, yellow fever, infectious ectromelia virus, Cowpox virus, infectious bovine rhinotracheitis virus, equine rhino-pneumonitis, (equine abortion) virus, malignant catarrh virus of cattle, feline rhinotracheitis virus, canine herpes virus, Epstein-Barr virus (associated with infectious mononucleosis and Burkitt lymphoma), Marek's disease virus, sheep pulmonary adenomatosis (Jaagziekte) virus, cytomegaloviruses, adenovirus group, human papilloma virus, feline panleucopaenia virus, mink enteritis virus, African horse sickness virus (9 serotypes), blue tongue virus (12 serotypes), infectious pancreatic necrosis virus of trout, fowl sarcoma virus (various strains), avian leukosis virus (visceral, etythroblastic and myeloblastic), osteopetrosis virus, Newcastle disease virus, parainfluenza virus 1, parainfluenza virus 2, parainfluenza virus 3, parainfluenza 4, mumps virus, turkey virus, CANADA/58, canine distemper virus, measles virus, respiratory syncytial virus, myxovirus, Type A viruses such as human influenza viruses, e.g., Ao/PR8/34, A1/CAM/46, and A2/Singapore/1/57; fowl plague virus; Type B influenza viruses, e.g., B/Lee/40, rabies virus, eastern equinine encephalitis virus, Venezuelan equine encephalitis virus, western equine encephalitis virus, yellow fever virus, Dengue type 1 virus (=type 6), Dengue type 2 virus (=type 5), Dengue type 3 virus, Dengue type 4 virus, Japanese encephalitis virus, Kyasanur Forest virus, Louping ill virus, Murray Valley encephalitis virus, Omsk haemorrhagic fever virus (types I and II), St. Louis encephalitis virus, human rhinoviruses, foot-and-mouth disease virus, poliovirus type 1, enterovirus polio 2, enterovirus polio 3, avian infectious bronchitis virus, human respiratory virus, transmissible gastro-enteritis virus of swine, lymphocytic choriomeningitis virus, lassa virus, Machupo virus, Pichinde virus Tacaribe virus, Papillomavirus, simian virus and Sindbis virus, and the like.

Peptide analogues of bacteria, for example, leprosy, tuberculosis, syphilis and gonorrhea, can also be used in the present invention.

Peptide analogues of the following parasites: organisms carrying malaria (P. Falciparum, P. Ovace, etc.), Schistosomiasis, Onchocerca Volvulus and other filiarial parasites, Trypanosomes, Leishmania, Chagas disease, amoebiasis, hookworm, and the like can also be utilized in the present invention.

The following peptides are non-limiting examples of peptides that can be utilized in the present invention:

A peptide containing an amino acid sequence mimicking the antigenic determinant of polyoma virus medium size tumor antigen is Lys-Arg-Ser-Ars-His-Phe (G. Walter, M.A. Hutchinson, T. Hunter and W. Eckhart, "Purification of Polyoma Virus Medium-Size Tumor Antigen by Immunoaffinity Chromatography", Proc. Natl. Acad. Sci USA, 79, 4025-4029, (1982)).

A peptide containing an amino acid sequence mimicking the antigenic determinant of poliovirus replicase antigen is as follows:

```
Tyr-Ser-Thr-Leu-Tyr-Arg-Arg-Trp-Leu-Asp-Ser-Phe
450                                          461
```

(M. H. Baron and D. Baltimore, "Antibodies Against a Synthetic Peptide of the Poliovirus Replicase Protein: Reaction with Native, Virus-Encoded Proteins and Inhibition of Virus-Specific Polymerase Activities In Vitro", Jour. Virology, 43, 3969-3978, (1982)).

Peptides containing an amino acid sequence mimicking the antigenic determinant of simian virus 40 large tumor antigen are as follows:

Met-Asp-Lys-Val-Leu-Asn-Arg and

Lys-Pro-Pro-Thr-Pro-Pro-Pro-Glu-Pro-Glu-Thr

(G. Walter, K.H. Scheidtmann, A. Carbone, A.P. Laudano, R.A. Lerner, N. Green, H. Alexander, F.-T. Liu, J.G. Sutcliffe and T.M. Shinnick, "Chemically Synthesized Peptides Predicted From the Nucleotide Sequence of the Hepatitis B Virus Genome Elicit Antibodies Reactive With the Native Envelope Protein of Dane Particles", Proc. Natl. Acad. Sci. USA, 78, 6, 3403-3407, (1981)).

A peptide containing an amino acid sequence mimicking the antigenic determinant of retrovirus R antigen is as follows:

Leu-Thr-Gln-Gln-Phe-His-Gln-Leu-Lys-Pro

Ile-Gly-Cys-Glu-Pro

(J.G. Sutcliffe, T.M. Shinnick, N. Green, F.-T. Liu, H.L. Niman and R.A. Lerner, "Chemical Synthesis of A Polypeptide Predicted From Nucleotide Sequence Allows Detection Of A New Retroviral Gene Product", Nature, 287, (1980)).

A peptide containing an amino acid sequence mimicking the antigenic determinant of avian sarcoma virus antigen is as follows:

Glu-Asp-Asn-Glu-Tyr-Thr-Ala-Arg-Gln-Gly,

(T.W. Wong and Alan R. Goldberg, "Synthetic Peptide Fragment Of src Gene Product Inhibits the src Protein Kinase and Cross Reacts Immunologically With Avian onc Kinases and Cellular Phosphoproteins", Proc. Natl. Acad. USA, 78, 12, 7412-7416, (1981)).

Peptides containing an amino acid sequence mimicking the antigenic determinant of foot-and-mouth disease virus antigen are as follows:

```
141
Val  Pro  Asn  Leu  Arg  Gly  Asp  Leu  Gly  Val

                                               160
Leu  Ala  Gly  Lys  Val  Ala  Arg  Thr  Leu  Pro

                     and

201
His  Lys  Gln  Lys  Ile  Val  Ala  Pro  Val  Lys  Gln
Thr  Leu,
```

(J.L. Bittle, R.A. Houghten, H. Alexander, T.M. Shinnick, J.G. Sutcliffe, R.A. Lerner, D.J. Rowlands and F. Brown, "Protection Against Foot-And-Mouth Disease By Immunization With A Chemically Synthesized Peptide Predicted From the Viral Nucleotide Sequence", Nature, 298, 30-33, (1982)).

A peptide containing an amino acid sequence mimicking the antigenic determinant of hemagglutinin X-31 (H3N2) influenza virus antigen is as follows:

```
123       125
Glu-Gly-Phe-Thr-Trp-Thr-Gly-

130                   135
Val-Thr-Gln-Asn-Gly-Gly-Ser-

            140
Asp-Ala-Cys-Lys-Arg-Gly-Pro-

        145               150
Gly-Ser-Gly-Phe-Phe-Ser-Arg-
151
Leu
```

(D.C. Jackson, J.M. Murray, D.O. White, C.N. Fagan and G.W. Tregear, "Antigenic Activity of a Synthetic Peptide Comprising the 'Loop' Region of Influenza Virus Hemagglutinin", Virology, 120, 273-276, (1982)).

A peptide containing an amino acid sequence mimicking the antigenic determinant of hemagglutinin of type A H3N2 influenza virus antigen was synthesized by G.M. Muller, M. Shapira and R.F. Doolittle, "Antibodies Specific for the Carboxy- And Amino- Terminal Regions of Simian Virus 40 Large Tumor Antigen", Proc. Natl. Acad. Sci USA, 77, 9, 5179-5200, (1980).

A peptide containing an amino acid sequence mimicking the antigenic determinant of influenza virus strain 3QB antigen is $Ile_1$ $Val_1$ $Asx_2$ $Thr_1$ $Ser_2$ $Glx_2$ $Pro_1$ $Glt_3$ $Ala_1$ $Leu_1$ $Lys_1$ (A. Aitken and C. Hannoun, "Purification of Haemagglutinin and Neuraminidase from Influenza Virus Strain 3QB and Isolation of a Peptide From an Antigenic Region of Haemagluttinin", Eur. J. Biochem., 107, 51-56, (1980)).

Peptides containing an amino acid sequence mimicking the antigenic determinant of diptheria antigen are as follows:

Natural DT Loop

```
-Cys-Ala-Gly-Asn-Arg-Val-Arg-Arg-Ser-Val-
  186               190                   195

Gly-Ser-Ser-Leu-Lys-Cys-
                    201
```

Synthetic peptide

| Tetradecapeptide | Gly(188)---Cys-(201) |
|---|---|
| Hexadecapeptide | Cys(186)---Cys-(201) |
| Octadecapeptide | Ala-Ala-Cys(186)---Cys-(201) |

(F. Audibert, M. Jolivet, L. Chedid, R. Arnon and M. Sela, "Successful Immunization With a Totally Synthetic Diphtheria Vaccine", Proc. Natl. Acad. Sci. USA, 79, 5042-5046, (1982)).

A peptide containing an amino acid sequence mimicking the antigenic determinant of Streptococcus pyogenes M antigen is as follows:

```
                        5
    Asn-Phe-Ser-Thr-Ala-Asp-Ser-Ala-Lys

    10                      15
    Ile-Lys-Thr-Leu-Glu-Ala-Glu-Lys-Ala-Ala-

    20                      25
    Leu-Ala-Ala-Arg-Lys-Ala-Asp-Leu-Glu-Lys-

    30                      35
    Ala-Leu-Glu-Gly-Ala-Met
```

(E.H. Beachey, J.M. Seyer, D.B. Dale, W.A. Simpson and A.H. Kang, "Type-Specific Protective Immunity Evoked by Synthetic Peptide of Streptococcus Pyogenes M Protein", Nature, 292, 457-459, (1981)).

The chemical synthesis of peptides (to produce "synthetic peptides") is described in the following publications: S.B.H. Kent, Biomedical Polymers, eds. Goldberg, E.P. and Nakajima, A. (Academic Press, New York), 213-242, (1980); A.R. Mitchell, S.B.H. Kent, M. Engelhard, and R.B. Merrifield, J. Org. Chem., 43, 2845-2852, (1978); J.P. Tam, T.-W. Wong, M. Riemen, F.-S. Tjoeng, and R.B. Merrifield, Tet. Letters, 4033-4036, (1979); S. Mojsov, A.R. Mitchell, and R.B. Merrifield, J. Org. Chem., 45, 555-560, (1980); J.P. Tam, R.D. DiMarchi and R.B. Merrifield, Tet. Letters, 2851-2854, (1981); and S.B.H. Kent, M. Riemen, M. Le Doux and R.B. Merrifield, Proceedings of the IV International Symposium on Methods of Protein Sequence Analysis, (Brookhaven Press, Brookhaven, N.Y.), in press, 1981.

Chemical Synthesis: In the so-called "Merrifield solid phase procedure" the appropriate sequence of L-amino acids is built up from the carboxyl terminal amino acid to the amino terminal amino acid. Starting with the appropriate carboxyl terminal amino acid attached to a polystyrene (or other appropriate) resin via chemical linkage to a chloromethyl group, benzhydrylamine group, or other reactive group of the resin, amino acids are added one by one using the following procedure. The peptide-resin is:

(a) washed with methylene chloride;

(b) neutralized by mixing for 10 minutes at room temperature with 5% (v/v) diisopropylethylamine (or other hindered base) in methylene chloride;

(c) washed with methylene chloride;

(d) an amount of amino acid equal to six times the molar amount of the growing peptide chain is activated by combining it with one-half as many moles of a carbodiimide (e.g., dicyclohexylcarbodiimide, or diisopropylcarbodiimide) for ten minutes at 0°C, to form the symmetric anhydride of the amino acid. The amino acid used should be provided originally as the N-alpha-tert.butyl oxcycarbonyl derivative, with side chains protected with benzyl esters (e.g., aspartic or glutamic acids), benzyl ethers (e.g., serine, threonine, cysteine or tyrosine), benzyloxycarbonyl groups (e.g., lysine) or other protecting groups commonly used in peptide synthesis.

(e) the activated amino acid is reacted with the peptide-resin for two hours at room temperature, resulting in addition of the new amino acid to the end of the growing peptide chain.

(f) the peptide-resin is washed with methylene chloride;

(g) the N-alpha-(tert. butyloxycarbonyl) group is removed from the most recently added amino acid by reacting it with 30 to 65%, preferably 50% (v/v) trifluoroacetic acid in methylene chloride for 10 to 30 minutes at room temperature;

(h) the peptide-resin is washed with methylene chloride; and

(i) steps (a) through (h) are repeated until the required peptide sequence has been constructed.

The peptide is then removed from the resin and simultaneously the side-chain protecting groups are removed by reaction with anhydrous hydrofluoric acid containing 10% v/v of anisole or other suitable (aromatic) scavenger. Subsequently, the peptide can be purified by gel filtration, ion exchange, high pressure liquid chromatography, or other suitable means.

In some cases, chemical synthesis can be carried out without the solid phase resin, in which case the synthetic reactions are performed entirely in solution. The reactions are similar and well known in the art, and the final product is essentially identical.

Isolation from natural sources: If sufficient quantities of the whole protein antigen are available, a limited portion of the molecule, bearing the desired sequence of amino acids may be excised by any of the following procedures:

(a) Digestion of the protein by proteolytic enzymes, especially those enzymes whose substrate specificity results in cleavage of the protein at sites immediately adjacent to the desired sequence of amino acids;

(b) Cleavage of the protein by chemical means. Particular bonds between amino acids can be cleaved by reaction with specific reagents. Examples include: bonds involving methionine are cleaved by cyanogen bromide; asparaginyl-glycine bonds are cleaved by hydroxylamine;

(c) A combination of proteolytic and chemical cleavages.

It should also be possible to clone a small portion of the DNA, either from natural sources or prepared by synthetic procedures, or by methods involving a combination thereof, that codes for the desired sequence of amino acids, resulting in the production of the peptide by bacteria, or other cells.

The polysaccharide carrier of the present invention is a high molecular weight material (preferably the molecular weight is at least 50,000) such as, for example, a dextran, e.g., Dextran 2000 (molecular weight of $2 \times 10^6$) and Ficoll 400 (molecular weight approximately 400,000). Dextran is a polymerized glucose in which the glucose units are joined through 1,6-glucoside links. "FICOLL" is an inert, non-ionized synthetic, high polymer made by a cross-linking reaction of epichlorohydrin and sucrose. It contains no ionized groups and is highly soluble due to its high content of hydroxyl groups (23%).

Amino groups can be introduced into the polysaccharides for use in the invention according to the method of John K. Inman, "Thymus Independent Antigens: The Preparation of Covalent Hapten-Ficoll Conjugates", Journal of Immunology, Vol. 114, pp. 704 to 709, (1975).

Peptides can be linked to the amino derivative of the polysaccharide carrier via an aliphatic dialdehyde (crosslinking agent) having 3 to 18 carbon atoms, preferably 4 to 6 carbon atoms either directly or through a diamino bridge (using amino acids with at least two amino groups, for example, lysine or a polyamino acid, for example, polylysine, polylysine-tyrosine or polylysine-polytyrosine). A preferred aliphatic dialdehyde for use in the present invention is glutaraldehyde. Alternatively, synthetic peptides with cysteine residues can be linked to the amino derivatives of the polysaccharides using heterobifunctional crosslinking agents.

Non-limiting examples of heterobifunctional crosslinking agents for use in the present invention include the following: m-maleimidobenzoyl-N-hydroxy-succinimide ester, N-succinimidyl (4-iodoacetyl) amino-benzoate, succinimidyl 4-(N-maleimidomethyl)cyclohexane-1-carboxylate, succinimidyl 4-(p-maleimidophenyl)-butyrate and N-succinimidyl-3-(2-pyridyldithio)propionate.

The resulting peptide linked to polysaccharide having multiple residues for saccharide molecule is quantatively adsorbed to a physiologically acceptable adjuvant such as, for example, alum.

Typical conditions for adsorption of polysaccharide-peptide conjugates to alum are as follows:

0.2 volumes of 0.2 M $KAl(SO_4)_2 \cdot 12H_2O$ are added to 1 volume of conjugate (containing 1 to 2 mg of derivatized (linked) peptide per ml). The pH is adjusted to 5.0 stepwise with 1 N l NaOH (within two hours at 20°C, incubation at pH 5.0). Then, 1 volume of 0.15 M NaCl - 0.01 M phosphate pH 6.2 is added and mixed with the suspension.

Alternatively, preformed alum can be added to the polysaccharide-peptide conjugate, so that the final concentration will be the same as given above. However, precipitation of alum from soluble $KAl(SO_4)_2 \cdot 12H_2O$ in the presence of the polysaccharide-peptide conjugate is preferable.

The resulting complexes are immunogenic and may be utilized as vaccines applicable for human use. The immunogenicity of the peptide-polysaccharide derivatives may also be enhanced by physiologically acceptable adjuvants other than alum.

A vaccine according to the present invention would comprise an immunologically effective amount of a complex according to the present invention, e.g., synthetic peptide linked to polysaccharide carrier adsorbed on alum, with a physiologically acceptable diluent medium, e.g., phosphate buffered saline. Generally speaking, the synthetic peptide concentration in a physiologically acceptable medium will be between approximately less than 1 milligram and more than 10 micrograms per dose.

The vaccine can be administered by subcutaneous, intradermal or intramuscular injection. While the preferred route would depend upon the particular vaccine, it is believed that intramuscular injection will be generally suitable. Frequency of administration will vary depending upon the vaccine. Generally speaking, the vaccine will be administered in two doses about one month apart followed by a booster at six months to one year after primary immunization. The subsequent doses or the booster will depend on the level of antibody in the blood as a result of the initial immunization and in certain instances may be unnecessary.

The invention will now be described with reference to the following non-limiting examples.

Examples

Example 1: Preparation of Amino-Dextran-Peptide Using the Heterobifunctional Agent SPDP

Amino groups were introduced into polysaccharides (Dextran 2000, Ficoll 400) by the method of Inman (J.K. Inman, "Thymus Independent Antigens: The Preparation of Covalent Hapten-Ficoll Conjugates", Journal of Immunology, Vol 114, p. 704, (1975)). 12 mg of $NH_2$ - Dextran in 0.1 M NaCl, 0.1 M phosphate pH 9 were mixed with 4 mg of N-succinimidyl-3-(2-pyridyldithio) propionate (SPDP) dissolved in a minimum volume of dimethylsulfoxide. The mixture was maintained for one hour at 23° C and the Dextran derivative was separated from excess of unreacted SPDP by gel-filtration on SEPHADEX G-25. The activated Dextran recovered in the void volume fractions after gel-filtration was mixed with 12 mg of either synthetic peptide hepatitis B preS(120-153) or preS(15-47), each having GGC residues at the C-terminus. The mixture was maintained overnight at 25° C. The optical density measured at 343 nm increased from an initial value of 0.234 to 1.9-2.05, indicating that the peptide became coupled to the Dextran derivative (according to instructions for SPDP provided by the manufacturer, Pharmacia, Upsala, Sweden). The peptide-Dextran conjugate was separated from side products and residual free synthetic peptide by molecular exclusion chromatography on SEPHADEX G-25. Analyses of the fractions indicated that more than 95% of each of the synthetic peptides became linked to the high molecular weight Dextran by this method.

Example 2: Preparation of Amino-Dextran-Peptide Conjugates Using The Heterobifunctional Agent Sulfo-MBS

Eight mg of the amino-Dextran derivative dissolved in 10 mM phosphate pH 8.5 were mixed with 3.5 mg of m-maleimidobenzoylsulfosuccinimide ester (sulfo-MBS). The reagent dissolved in water was added dropwise within one hour at 23° C. The derivatized amino-dextran was separated from excess of unreacted reagent by gel-filtration on SEPHADEX G-25 in 0.1M phosphate pH 6.5. Fractions corresponding to the void volume were mixed with 8 mg of either hepatitis B peptide preS(120-153) or preS(15-47) (each of these peptides had glycine-glycine-cysteine (GGC) residues added at the C-terminus to allow the coupling to the dextran derivative through the C-terminal cysteine residues). The Dextran-peptide mixtures were maintained overnight at room temperature and then subjected to molecular exclusion chromatography on SEPHADEX G-50 in order to separate the peptide-Dextran derivatives from excess reagents and unreacted peptides.

Example 3: Adsorbtion of Synthetic Peptide-Dextran Conjugates to Alum

One volume of peptide Dextran derivatives prepared as described in Examples 1 and 2, or as described in examples listed below, was mixed with 0.3 volumes of 0.2 M potassium aluminum sulfate. The pH of the mixture was adjusted to 5.0 by addition of 1N NaOH. The mixture was maintained at 20° C for two hours, maintaining the pH at 5.0, then one volume of 0.15 M NaCl, 0.01 M phosphate pH 6.2 was added. An aliquot of the resulting suspension was centrifuged and both the supernate and the Dextran derivative before addition of alum were analyzed for the presence of antigenic determinants corresponding to the respective synthetic peptides. For this purpose, polystyrene beads were coated with serial 10-fold dilutions of the respective preparations for at least two hours at room temperature. The unreacted components were removed. The beads were post-coated with a solution containing 1% bovine serum albumin and 0.25% gelatin and incubated for at least one hour at 20° C. The excess proteins were removed, the beads were washed and, finally, tested for antigenic determinants corresponding to the synthetic peptide by double antibody radioimmunoassays utilizing rabbit antibodies to the respective synthetic peptides in the first step and $^{125}$I-labeled anti-rabbit IgG in the second step. The results revealed that 80-99% of the peptide Dextran derivatives became attached to alum.

Example 4: Preparation of Synthetic Peptide-Dextran Derivatives Based on the Linking of Peptides to Glutaraldehyde Polymerized (Crosslinked) Amino-Dextran

Amino-Dextran was treated with 1M glutaraldehyde (GA) in 0.1 M phosphate at pH 8.5 for at least two hours at 20° C. The resulting polymer was separated from excess glutaraldehyde either by gel-filtration on SEPHADEX G-25 or by extensive dialysis against 0.1 M phosphate pH 8.5. The Dextran polymer containing aldehyde groups (GA-dextran) can be used directly for coupling of peptides through their available amino groups. Alternatively, the aldehyde derivatives can be reacted with cysteine. This reaction involves the aldehyde groups of the polymer and the amino groups of cysteine resulting in a polymer with active SH groups which can be utilized to link synthetic peptides have cysteine residues. Alternatively, the polymer with aldehyde groups can be reacted with lysine, polylysine, polylysine-polytyrosine and so on, resulting in polymers having both amino and carboxyl groups available for the linking of peptides.

Example 5: Direct Linking of Peptides to Glutaraldehyde-activated Amino-Dextran

Two mg aliquots of glutaraldehyde amino-dextran were mixed with 4 mg of hepatitis B synthetic peptides preS(120-153)adw₂GGC and preS(15-47)ayw, respectively. The mixtures were maintained at 4°C overnight and then subjected to molecular exclusion chromatography on SEPHADEX G-25. The peptide became quantitatively linked to the Dextran derivative and was recovered in the void volume of the column.

Example 6: Preparation of Lysine-Glutaraldehyde Amino-Dextran Synthetic Peptide Derivatives

150 mg of amino-dextran (concentration: 10 mg per ml) were treated with 1M glutaraldehyde as described above. The resulting derivative, after removal of excess glutaraldehyde by dialysis, was treated with lysine hydrochloride at pH 8.5. The amount of lysine corresponded to 15 ml of a 1M solution. The mixture was maintained for five hours at 20°C and then dialyzed excessively against 10 mM phosphate pH 8.5. An aliquot of the resulting product (corresponding to 8 mg of dextran) was derivatized with 3.5 mg of MBS-SO₃ as described above. The product was separated from excess of MBS-SO₃ by molecular exclusion chromatography on SEPHADEX G-25 and mixed with 8 mg portions of either hepatitis B peptide preS(120-153)GGC or preS(15-47)GGC in 1M phosphate pH 6.5. The resulting peptide-dextran derivative was isolated by gel-filtration on SEPHADEX G-50 and was recovered in the void volume fractions of the column.

Example7: Polylysine (Polylysine-Tyrosine)-Glutaraldehyde Activated Amino-Dextran Derivatives

Two mg quantities of either polylysine or polylysine-tyrosine (the molecular weights of the polymers were in the range of 20 to 32 KDa) were dissolved in 0.1M phosphate, 0.1M NaCl pH 9 and reacted with 300μg of SPDP dissolved in a minimum volume of ethanol. After incubating at 23°C, the derivatized polymers were separated from excess SPDP by gel-filtration on SEPHADEX G-10 in 0.1M phosphate, 0.1M NaCl, pH 8. The void volume fractions were reacted with 2 mg of reduced hepatitis B peptides preS(120-153)GGC and preS(14-47)GGC, respectively. The mixtures were maintained overnight at 25°C and subjected to gel-filtration on SEPHADEX G-25 in 0.05 M phosphate pH 6. The void volume fractions were mixed with 4 mg of GA-dextran. After overnight incubation at 20°C the mixtures were subjected to gel-filtration on SEPHADEX 6 BCL. Fractions corresponding to the void volume were adsorbed on alum as described in Example 3 hereinabove.

Example 8: Effect of Different Carriers on the Immunogenicity of Hepatitis B PreS2 and PreS1 Peptides

The results of this example are depicted in Fig. 1.
Alum was used as an adjuvant for all synthetic peptide-dextran derivatives depicted in Fig. 1. In the case of GA-dextran derivatives, rabbits immunized with five 100-200 μg doses of immunogens with alum adjuvants were further hyperimmunized with the same immunogens using two additional doses with an adjuvant from Syntex, California, U.S.A., instead of alum. This adjuvant is based on pluronic acids. The results on the graph of Fig. 1 correspond to the antisera obtained after the final bleeding of rabbits. Hyperimmunization with immunogens in combination with the Syntex adjuvant resulted in a further 2- to 5-fold increase of antibody levels as compared with the last bleedings obtained after immunization with the respective immunogens in combination with alum. Two rabbits were used per immunogen; geometric mean antibody titers were plotted.

Results

Surprisingly, polymers prepared by reacting peptides with activated dextran (without using glutaraldehyde as a linker, i.e., uncrosslinked dextran), as well as similar derivatives based on Ficoll had all extremely low immunogenicities when alum was used as adjuvant. However, derivatives based on crosslinked dextran (GA-dextran) elicited antibodies recognizing not only the free synthetic peptides, but also preS sequences within the native hepatitis B surface antigen. See Fig. 1.

It will be appreciated that the instant specification and claims are set forth by way of illustration and not limitation and that various modifications and changes may be made without departing from the spirit and scope of the present invention.

**Claims**

1. A physiologically acceptable immunogenic complex comprising a peptide linked to a non-immunogenic, high molecular weight polysaccharide carrier, said carrier having been converted into an amino derivative, said peptide linked to said carrier being adsorbed onto a physiologically acceptable adjuvant.

2. An immunogenic complex according to claim 1, wherein the amino-derivaive of the polysaccharide is cross-linked with an aliphatic dialdehyde which has preferably 3 to 18, more preferably 4 to 6 carbon atoms and is most preferably glutaraldehyde.

3. An immunogenic complex according to claims 1 or 2, wherein the peptide contains cysteine residues which are linked to amino derivatives of the polysaccharide using a heterobifunctional cross-linking agent.

4. An immunogenic complex according to anyone of claims 1-3, wherein the adjuvant comprises alum, optionally together with one or moe physiologically acceptable adjuvants other than alum.

5. An immunogenic complex according to anyone of claims 1-4, wherein a diamino bridge for linking the peptide to the polysaccharide is formed by using amino acids or polyamino acids with at least two amino groups, said amino acids or polyamino acids being preferably selected from the group consisting of lysine, polylysine and polylysine-tyrosine.

6. An immunogenic complex according to anyone of claims 1-5, wherein the peptide is a hepatitis B virus preS peptide.

7. An immunogenic complex according to anyone of claims 1-5, wherein the peptide is chemically synthesized, cleaved from a natural material or produced by recombinant DNA techniques.

8. A vaccine comprising an immunogenically effective amount of an immunogenic complex according to anyone of claims 1-5 in a physiologically acceptable diluent.

9. A method for preparing the physiologically acceptable immunogenic complex according to anyone of claims 1-7 comprising

(a) introducing amino groups onto a high molecular weight, non-immunogenic polysaccharide carrier,

(b) linking a peptide to the high molecular weight polysaccharide carrier containing amino groups of step (a) and (c) adsorbing the peptide linked to the carrier of step (b) onto a physiologically acceptable adjuvant.

10. A method according to claim 9, further comprising contacting the product of step (a) with an aliphatic dialdehyde to introduce aldehyde groups on the carrier.

11. A method according to claims 9 or 10 comprising reacting the aldehyde-containing carrier with cysteine, or with amino acids or polyamino acids having at least two amino groups, preferably with lysine, polylysine and/or polylysine-polytyrosine.

FIG. 1